# EUROPÄISCHE PATENTSCHRIFT

(11) **EP 0 856 323 B1**
(45) Veröffentlichungstag und Bekanntmachung des Hinweises auf die Patenterteilung: **11.12.2002**
(21) Anmeldenummer: 98101669.4
(22) Anmeldetag: 31.01.1998
(51) Int. Cl.: A61M 5/142

(54) **Vorrichtung zur Dosierung von medizinischen Flüssigkeiten**
Medical liquid metering device
Dispositif pour le dosage des fluides médicaux

(30) Priorität: 04.02.1997 DE 29701861 U
(43) Veröffentlichungstag der Anmeldung: 05.08.1998
(73) Patentinhaber: Fresenius AG, 61352 Bad Homburg v.d.H. (DE)
(72) Erfinder: Schäfer, Helmut, 61250 Usingen (DE)
(74) Vertreter: Luderschmidt, Schüler & Partner GbR

(56) Entgegenhaltungen:
- EP-A- 0 193 266
- EP-A- 0 296 124
- WO-A-97/45150
- DE-B- 1 026 047
- US-A- 4 634 431

## Beschreibung

Die Erfindung betrifft eine Vorrichtung zur Dosierung von medizinischen Flüssigkeiten, insbesondere einer Nährlösung zur enteralen Ernährung. Darüber hinaus bezieht sich die Erfindung auf eine Anordnung zur enteralen Ernährung mit einer derartigen Vorrichtung zur Dosierung der Nährlösung.

Eine Vorrichtung zur dosierten Abgabe einer medizinischen Flüssigkeit, beispielsweise eines Medikaments, ist aus der WO 93/14797 bekannt. Die Medikamentenpumpe weist ein Gehäuse auf, das aus zwei miteinander verschraubten schalenförmigen Elementen besteht, die einen kollabierbaren Behälter aufnehmen, der mit der medizinischen Flüssigkeit gefüllt ist. In dem Gehäuse wird der Flüssigkeitsbeutel von einer federnd vorgespannten Platte mit Druck beaufschlagt, so daß die Flüssigkeit über eine Schlauchleitung aus dem Behälter austritt.

Zur Behandlung von Patienten mit Mangelernährung ist es bekannt, eine Nährlösung aus einem Behälter über einen Schlauch und eine im Gastroindestinaltrakt plazierte Sonde dem Patienten zu applizieren. Für Langzeitpatienten finden ambulante Ernährungssysteme Verwendung, mit denen sich der Patient im wesentlichen frei bewegen kann, abgesehen von der Tatsache, daß er sämtliche für die ambulante Versorgung notwendigen Geräte mit sich führen muß. Zu diesen Geräten gehört der die Nährlösung enthaltende Behälter, der über ein Anschlußstück für das Überleitgerät zum Zuführen der Nährlösung verfügt, das Überleitgerät und eine peristaltische Pumpe zum kontinuierlichen Fördern der Nährlösung mit einer vorgegebenen Förderrate, in die der Ernährungsschlauch des Überleitgeräts eingelegt wird. Der Vorteil der Schlauchpumpe liegt darin, daß die medizinische Flüssigkeit nicht mit den Bauteilen der Pumpe in Verbindung kommt.

Die bekannten Ernährungssysteme haben sich in der Praxis bewährt, nachteilig ist jedoch, daß die Schlauchpumpe aufgrund ihrer bauartbedingten Größe und des relativ hohen Gewichts während der ambulanten Behandlung vom Patienten als störend empfunden wird. Darüber hinaus ist die Herstellung der Schlauchpumpe mit relativ hohen Kosten verbunden.

Die EP-A-0 193 266 beschreibt eine Vorrichtung zur Dosierung von medizinischen Flüssigkeiten mit zwei Gehäuseteilen, von denen das eine Gehäuseteil eine Pumpenkammer mit Ein- und Auslass und einem Verdrängerelement und das andere Gehäuseteil einen Antrieb für den Verdränger der Pumpenkammer umfasst. Beide Gehäuseteile können zusammengesetzt und mittels verschiedener Kupplungselemente miteinander verbunden werden. Nachteilig ist, dass die verschiedenen Kupplungselemente die Handhabung der Dosiervorrichtung erschweren.

Eine Antriebseinheit für eine Spritzenpumpe , die einen mit einer aufziehbaren Spiralfeder betriebenen Motor aufweist, ist aus der US-A-4,634,431 bekannt.

Der Erfindung liegt die Aufgabe zugrunde, eine Vorrichtung zur Dosierung von medizinischen Flüssigkeiten, insbesondere einer Nährlösung zur enteralen Ernährung, mit einer kompakten Bauart zu schaffen, die sich einfach handhaben und kostengünstig betreiben läßt.

Die Lösung der Aufgabe erfolgt erfindungsgemäß mit den im Schutzanspruch 1 angegebenen Merkmalen.

Die erfindungsgemäße Vorrichtung zum Dosieren von medizinischen Flüssigkeiten ist nicht nach Art einer Schlauchpumpe ausgebildet. Die Förderung der medizinischen Flüssigkeit erfolgt mittels eines Verdrängers, der in einer Pumpenkammer angeordnet ist. Obwohl die medizinische Flüssigkeit mit Bauteilen der Dosiervorrichtung in Verbindung kommt, ist eine aufwendige Reinigung der flüssigkeitsführenden Teile nicht erforderlich, da sich die mit der Flüssigheit in Verbindung kommenden Teile nach dem einmaligen Gebrauch der Vorrichtung einfach austauschen lassen. Diese bilden einen Einwegartikel, der in großen Stückzahlen kostengünstig hergestellt werden kann. Die in der Herstellung relativ kostspielige Antriebseinheit des Verdrängers ist hingegen zur Wiederverwendung bestimmt. Der Verdränger kann beispielsweise als Ümlaufverdränger oder Hubverdränger ausgebildet sein.

Die erfindungsgemäße Dosiervorrichtung weist zwei voneinander trennbare Gehäuseteile auf, von denen der eine Teil die Pumpenkammer mit dem Verdränger und der andere Teil die Antriebseinheit des Verdrängers aufnimmt. Die Übertragung des Drehmoments erfolgt mittels Kupplungselementen, die beim Ankoppeln der Antriebseinheit in Eingriff kommen.

Die beiden Gehäuseteile sind miteinander verschraubbar. Zur Verbindung der Gehäuseteile kann z.B. ein Bajonettverschluß vorgesehen sein.

In einer bevorzugten Ausführungsform weist die Antriebseinheit einen mit einer aufziehbaren Spiralfeder angetriebenen Motor auf. Damit kann die Dosiervorrichtung unabhängig vom elektrischen Netz oder von Batterien betrieben werden, so daß diese jederzeit einsatzbereit ist. Es ist aber auch möglich, den Verdränger mit einem batteriebetriebenen Elektromotor anzutreiben.

Um die Förderrate variieren zu können, weist die Antriebseinheit vorzugsweise ein Getriebe mit einem einstellbaren Übersetzungsverhältnis auf. Das Getriebe kann aber auch als auswechselbares Modul ausgebildet sein, das sich zum Vorgeben einer kleineren oder größeren Förderrate gegen ein Getriebe mit einem anderen Übersetzungsverhältnis austauschen läßt.

In einer vorteilhaften Ausgestaltung bildet die Abtriebswelle der Antriebseinheit beim Ankoppeln derselben eine formschlüssige Verbindung mit dem Verdränger. Der Verdränger weist vorteilhafterweise eine zentrale Ausnehmung mit einer Innenkontur auf, während die in die Ausnehmung greifende Abtriebswelle mit einer entsprechenden Außenkontur versehen ist.

Die Dosiervorrichtung findet in vorteilhafter Weise zur Dosierung einer aus einem Behälter über einen Ernährungsschlauch einer Sonde zugeführten Nährlösung Verwendung, die einen in den Magen des Patienten einzuführenden Sondenschlauch aufweist. Dabei wird die Dosiervorrichtung in den Ernährungsschlauch geschaltet. Zum Anschluß der Schlauchenden sind an der Dosiervorrichtung zweckmäßigerweise Konnektoren vorgesehen, z.B. die in der Medizintechnik bekannten Luer-Lock-Konnektoren oder Trichteranschlüsse, über die auch die herkömmlichen Schlauchsysteme verfügen.

Gegenüber den bekannten Systemen zur enteralen Ernährung, bei denen die Förderung der Nährlösung allein durch die Gravitation erfolgt, hat eine Anordnung, in deren Ernährungsschlauch die erfindungsgemäße Dosiervorrichtung geschaltet ist, den Vorteil, daß die Förderrate von der Viskosität der Nährlösung und dem Füllvolumen des Fördergutdepots weitgehend unabhängig ist. Gegenüber den Ernährungssystemen mit den bekannten peristaltischen Pumpen besteht nicht die Gefahr, daß der Pumpenschlauch auf Dauer ermüdet, wodurch Veränderungen der Förderrate auftreten könnten. Ein weiterer Vorteil liegt in der kompakten Bauweise, die dem Patienten während der ambulanten Sondenernährung mehr Freiraum gibt. Darüber hinaus ist eine Spülung der Dosiervorrichtung nach dem Gebrauch nicht erforderlich, weil die Pumpeinheit als Einmalartikel auswechselbar ist. Da die Antriebseinheit wiederverwendbar ist, läßt sich die Dosiervorrichtung kostengünstig betreiben.

Im folgenden wird ein Ausführungsbeispiel der Erfindung unter Bezugnahme auf die Zeichnungen näher erläutert.

Es zeigen:
- Fig. 1: eine bevorzugte Ausführungsform der Vorrichtung zur Dosierung von medizinischen Flüssigkeiten in teilweise geschnittener Darstellung, wobei die Antriebseinheit von der Pumpeinheit abgeschraubt ist,
- Fig. 2: die Pumpeinheit der Dosiervorrichtung in der Draufsicht,
- Fig. 3: einen Schnitt durch die Pumpeinheit der Dosiervorichtung entlang der Linie III-III von Fig. 1,
- Fig. 4: den Verdränger der Pumpeinheit in teilweise geschnittener Darstellung. und
- Fig. 5: eine Anordnung zur enteralen Ernährung, in deren Ernährungschlauch die Dosiervorrichtung geschaltet ist.

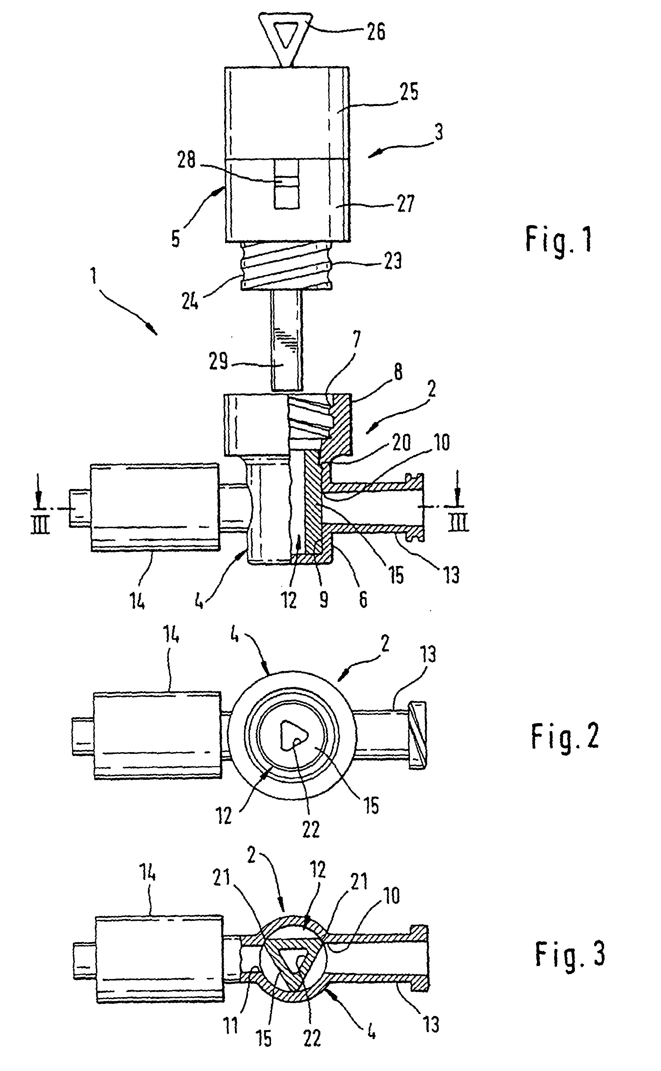

Fig. 1 zeigt die Dosiervorrichtung 1 in teilweise geschnittener Darstellung, die aus einer als Einwegartikel ausgebildeten Pumpeinheit 2 und einer zur Wiederverwendung bestimmten Antriebseinheit 3 besteht, wobei das Gehäuseteil 4 der Pumpeinheit und das Gehäuseteil 5 der Antriebseinheit miteinander verschraubt sind.

Das Gehäuseteil 4 der Pumpeinheit 2 umfaßt einen hohlzylindrischen Körper 6, der an einem Ende geschlossen und an dem anderen Ende mit einem ein Innengewinde 7 aufweisenden Befestigungsflansch 8 versehen ist. In der Zylinderwand 9 sind zwei umfangsmäßig um 180° versetzt zueinander angeordnete Bohrungen vorgesehen, wobei die mit der Bezugsziffer 10 bezeichnete Bohrung den Einlaß und die mit der Bezugsziffer 11 bezeichnete Bohrung den Auslaß der zylindrischen Pumpenkammer 12 bildet. Zum Anschluß eines Schlauchleitungssystems weist die Pumpeinheit 2 einen an dem Einlaß angeschlossenen Luer-Lock-Außenkegel 14 und einen an den Auslaß angeschlossenen Luer-Lock-Innenkegel 13 auf, die einstückiger Bestandteil des Gehäuseteils 4 sind. Die Luer-Lock-Konnektoren 13, 14 liegen auf einer gemeinsamen rechtwinklig zur Längsachse der Pumpenkammer 12 verlaufenden Achse.

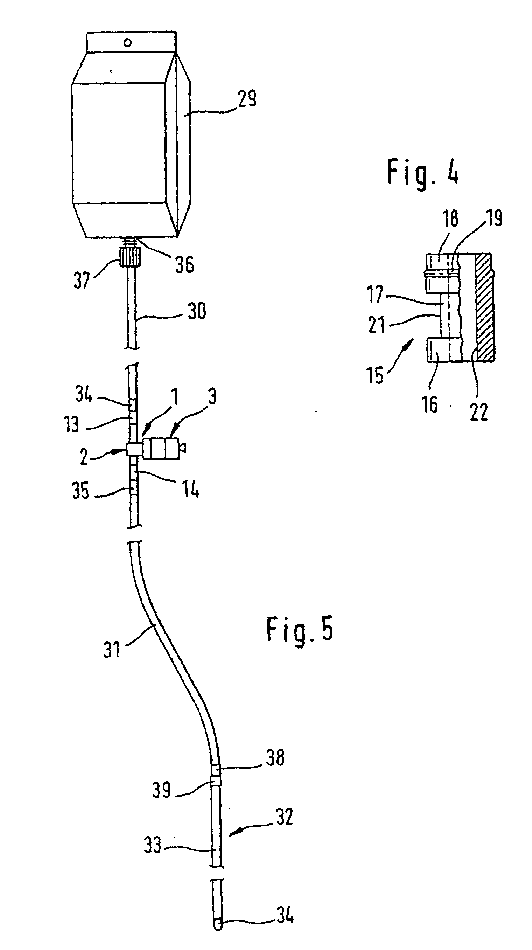

In der zylindrischen Pumpenkammer 12 des Gehäuseteils 4 sitzt ein während des Betriebs der Dosiervorrichtung rotierender Verdränger 15, der als Einsteckteil ausgebildet ist (Fig. 4). Der Verdränger 15 weist einen zylindrischen unteren Abschnitt 16, einen mittleren Abschnitt 17 mit im wesentlichen dreieckförmigen Querschnitt und einem zylindrischen oberen Abschnitt 18 auf. In der zylindrischen Pumpenkammer 12 ist der Verdränger 15 durch einen umlaufenden Absatz 19 an seinem oberen zylindrischen Abschnitt 18 gegen Herausrutschen gesichert, mit dem sich der Verdränger an einem umlaufenden Vorsprung 20 in der Zylinderwand 9 abstützt. Die abgerundeten Ecken 21 des mittleren Abschnitts 17 mit dem dreieckförmigen Querschnitt liegen dicht an der Zylinderwand 9 an. Der Verdränger 15 weist ferner eine axiale Ausnehmung 22 mit einer dreieckförmigen Kontur auf.

Das Gehäuseteil 5 der Antriebseinheit 3 weist einen zylindrischen Absatz 23 mit einem Außengewinde 24 auf, das sich mit dem Befestigungsflansch 8 der Pumpeinheit 2 verschrauben läßt. Es nimmt einen mit einer aufziehbaren Spiralfeder betriebenen Motor 25 auf. Es handelt sich hierbei um einen aus Uhrwerken oder dgl. bekannten Federmotor, der nur andeutungsweise dargestellt ist. Zum Aufziehen der Spiralfeder ist im Gehäusedeckel eine abklappbare Schraube 26 vorgesehen. Dem Motor 25 ist ein Getriebe 27 mit einem einstellbaren Übersetzungsverhältnis nachgeschaltet. Die Einstellung des Übersetzungsverhältnisses erfolgt mittels eines in dem Gehäuseteil 5 integrierten Schiebeschalters 28, wobei drei unterschiedliche Drehzahlen vorgegeben werden können. Die Abtriebswelle 29 des Getriebes 27 erstreckt sich durch den zylindrischen Gehäuseabsatz 23 und weist einen der axialen Ausnehmung 22 des Verdrängers 15 entsprechenden dreieckförmigen Querschnitt auf. Beim Aufschrauben der beiden Gehäuseteile 4, 5 greift die Abtriebswelle 22 in die Ausnehmung 22 des Verdrängers, so daß das Drehmoment auf den Verdränger übertragen werden kann.

Fig. 5 zeigt eine Anordnung zur enteralen Ernährung, bei der die Vorrichtung zur Dosierung der Nährlösung Verwendung findet. Die Anordnung umfaßt einen die Nährlösung enthaltenden Behälter 29, ein erstes Schlauchstück 30, die Dosiervorrichtung 1, ein zweites Schlauchstück 31 und eine Sonde 32, die einen in den Magen des Patienten einzuführenden Sondenschlauch 33 aufweist, dessen distales Ende mit einer endständig und/oder seitlich offenen Olive 34 versehen ist. Der Anschluß der Dosiervorrichtung 1 erfolgt mittels Luer-Lock-Konnektoren 34, 35 an den jeweiligen Schlauchenden, die mit den entsprechenden Luer-Lock-Anschlußstücken an der Dosiervorrichtung 1 verbunden sind. Der Behälter 29 weist ein bodenseitiges Anschlußstück 36 mit einer durchstechbaren Membran auf, die mit einem in dem Konnektor 37 des ersten Schlauchstücks 30 integrierten Spike durchstoßen wird. Die Verbindung des zweiten Schlauchstücks 31 mit der Emährungssonde 32 erfolgt mittels Luer-Lock-Konnektoren 38, 39.

Während des Betriebs ist die Dosiervorrichtung 1 unterhalb des die Nährlösung enthaltenden Beutels 29 angeordnet, so daß die Flüssigkeit aufgrund der Schwerkraft durch das erste Schlauchstück 30 in die Pumpkammer 12 der Dosiervorrichtung strömt. In Abhängigkeit von der Drehzahl des rotierenden Verdrängers 15 wird die Nährlösung über das zweite Schlauchstück in den Sondenschlauch 33 geleitet, wobei sich die Förderrate durch Einstellen des Übersetzungsverhältnisses verändern läßt.

Nach dem Gebrauch wird die Antriebseinheit 3 der Dosiervorrichtung 1 von der Pumpeinheit 2 abgeschraubt und die Luer-Lock-Konnektorverbindungen zwischen der Dosiervorrichtung und den Ernährungsschläuchen 30, 31 werden gelöst. Die von der Nährlösung durchströmte Pumpeinheit 2 kann dann gegen eine neue Einheit ausgetauscht werden, so daß eine Reinigung nicht erforderlich ist.

## Patentansprüche

1. Vorrichtung zur Dosierung von medizinischen Flüssigkeiten, insbesondere einer Nährlösung zur enteralen Ernährung, mit einem Gehäuseteil (2), der eine Pumpenkammer (1) mit einem Einlass (10) und Auslass (11) aufweist, in der ein die medizinische Flüssigkeit fördernder Verdränger (25, 27) angeordnet ist, und einem Gehäuseteil (3), der den Antrieb des Verdrängers aufnimmt, wobei der die Pumpenkammer (12) aufweisende Gehäuseteil (4) und der den Verdrängerantrieb (25) aufnehmende Gehäuseteil (5) voneinander trennbar sind, und der Verdränger (15) und dessen Antrieb Kupplungselemente (22, 29) aufweisen, die beim Aneinandersetzen der Gehäuseteile ineinandergreifen, so dass die Antriebseinheit (3) an die Pumpeneinheit (2) ankoppelbar bzw. von der Pumpeneinheit abnehmbar ist, **dadurch gekennzeichnet, dass** die beiden Gehäuseteile sellst (4, 5) als ineinander schraubbare Verbindungselemente ausgebildet sind.

2. Vorrichtung nach Anspruch 1, **dadurch gekennzeichnet, dass** die Antriebseinheit (3) einen mit einer aufziehbaren Spiralfeder betriebenen Motor (25) aufweist.

3. Vorrichtung nach Anspruch 1 oder 2, **dadurch gekennzeichnet, dass** die Antriebseinheit (3) ein Getriebe (27) aufweist, dessen Übersetzungsverhältnis einstellbar ist.

4. Vorrichtung nach einem der Ansprüche 1 bis 3, **dadurch gekennzeichnet, dass** die Pumpenkammer (12) im wesentlichen zylindrisch ist, wobei der Einlass (10) und Auslass (11) in der Zylinderwand (9) umfangsmäßig um 180° versetzt zueinander angeordnet sind, und dass der Verdränger (15) ein in der Pumpenkammer drehbarer Körper ist, der mehrere flügelartige an der Zylinderwand dicht anliegende Verdrängerelemente aufweist.

5. Vorrichtung nach Anspruch 4, **dadurch gekennzeichnet, dass** der Verdränger (15) eine zentrale Ausnehmung (22) mit einer Innenkontur und die Antriebseinheit (3) eine mit einer entsprechenden Außenkontur versehene Antriebswelle (29) aufweist, die beim Ankoppeln der Antriebseinheit in die Ausnehmung des Verdrängers greift.

6. Vorrichtung nach einem der Ansprüche 1 bis 5, **dadurch gekennzeichnet, dass** der die Pumpenkammer (12) aufnehmende Gehäuseteil (4) zwei Konnektoren (13, 14) zum Anschluss eines Ernährungsschlauchs an den Einlass (11) und Auslass (10) der Pumpenkammer aufweist.

7. Ernährungsschlauch zum Überleiten einer Nährlösung zur enteralen Ernährung von einem die Nährlösung enthaltenden Beutel (29) zu einer Sonde (32), die einen in den Magen des Patienten einzuführenden Sondenschlauch (33) aufweist, **gekennzeichnet durch** eine in den Ernährungsschlauch (30, 31) geschaltete Vorrichtung (1) zur Dosierung der Nährlösung nach einem der Ansprüche 1 bis 6.

8. Anordnung zur enteralen Ernährung mit einem die Nährlösung enthaltenden Beutel (29), einem Ernährungsschlauch (30, 31) und einer Sonde (32), die einen in den Magen des Patienten einzuführenden Sondenschlauch (33) aufweist, **gekennzeichnet durch** eine in den Ernährungsschlauch (30, 31) geschaltete Vorrichtung (1) zur Dosierung der Nährlösung nach einem der Ansprüche 1 bis 6.

## Claims

1. Device for metering medical fluids, particularly a nutrient solution for enteral feeding, with a housing section (2) which exhibits a pump chamber (1) with an inlet (10) and an outlet (11), in which chamber is arranged a compressor (25, 27) conveying the medical fluid, and with a housing section (3) which drives the compressor, in which the housing section (4) exhibiting the pump chamber (12) and the housing section (5) driving the compressor (25) may be separated from each other, and in which the compressor (15) and its drive exhibit coupling elements (22, 23) which engage in each other when the housing sections are assembled together, so that the drive unit (3) can be connected to the pump unit (2) and removed from it, **characterised in that** the two housing sections are themselves designed as connecting elements (4, 5) that can be screwed into each other.

2. Device according to claim 1, **characterised in that** the drive unit (3) exhibits a motor (25) operated with a helical spring that can be tightened.

3. Device according to claim 1 or 2, **characterised in that** the drive unit (3) exhibits a gear (27) whose reduction ratio is adjustable.

4. Device according to one of claims 1 to 3, **characterised in that** the pump chamber (12) is essentially cylindrical, the inlet (10) and outlet (11) being arranged in the cylinder wall (9) so that they are peripherally displaced by 180° to each other, and **in that** the compressor (15) is an element that can be rotated in the pump chamber and exhibits several wing-type compressor elements in close contact with the cylinder wall.

5. Device according to claim 4, **characterised in that** the compressor (15) exhibits a central recess (22) with an inner contour, and **in that** the drive unit (3) exhibits a drive shaft (29) provided with a corresponding outer contour, which shaft engages in the recess of the compressor when the drive unit is connected.

6. Device according to one of claims 1 to 5, **characterised in that** the housing section (4) receiving the pump chamber (12) exhibits two connectors (13, 14) for connecting a feeding hose to the inlet (11) and outlet (10) of the pump chamber.

7. Feeding hose for transferring a nutrient solution for enteral feeding from a bag (29) containing the nutrient solution to a probe (32) which exhibits a probe hose (33) to be introduced into the stomach of the patient, **characterised by** a device (1) connected to the feeding hose (30, 31) for metering the nutrient solution according to one of claims 1 to 6.

8. Device for enteral feeding, with a bag (20) containing the nutrient solution, a feeding hose (30, 31) and a probe (32), which exhibits a probe hose (33) to be introduced into the stomach of the patient, **characterised by** a device (1) connected to the feeding hose (30, 31) for metering the nutrient solution according to one of claims 1 to 6.

## Revendications

1. Dispositif pour le dosage de liquides médicaux, en particulier d'une solution nutritive pour l'alimentation entérale, avec une partie de boîtier (2), qui présente une chambre de pompe (1) avec une entrée (10) et une sortie (11), dans laquelle est disposé un déplaceur (25, 27) transportant le liquide médical, et une partie de boîtier (5), qui reçoit la commande du déplaceur, la partie de boîtier (4) présentant la chambre de pompe (12) et la partie de boîtier (5) recevant la commande de déplaceur (25) pouvant être séparées l'une de l'autre, et le déplaceur (15) et sa commande présentant des éléments de couplage (22, 29), qui s'emboîtent l'un dans l'autre lorsqu'on place des parties de boîtier l'une à côté de l'autre, de sorte que l'unité d'entraînement (3) peut être couplée à l'unité de pompage (2) et peut être enlevée de l'unité de pompage, **caractérisé en ce que** les deux parties de boîtier sont conçues même comme des éléments de liaison (4, 5) pouvant être vissés l'un dans l'autre.

2. Dispositif selon la revendication 1, **caractérisé en ce que** l'unité d'entraînement (3) présente un moteur (25) fonctionnant avec un ressort en spirale enroulable.

3. Dispositif selon la revendication 1 ou 2, **caractérisé en ce que** l'unité d'entraînement (3) présente une boîte de vitesses (27), dont le rapport de transmission est réglable.

4. Dispositif selon l'une quelconque des revendications 1 à 3, **caractérisé en ce que** la chambre de pompe (12) est sensiblement cylindrique, l'entrée (10) et la sortie (11) étant disposées dans la paroi de cylindre (9) à la périphérie de façon décalée de 180° l'une par rapport à l'autre, et **en ce que** le déplaceur (15) est un corps pouvant tourner dans la chambre de pompe, lequel présente plusieurs éléments refouleurs du type ailette et s'appuyant de façon étanche sur la paroi de cylindre.

5. Dispositif selon la revendication 4, **caractérisé en ce que** le déplaceur (15) présente un évidement (22) central avec un contour intérieur et l'unité d'entraînement (3) un arbre d'entraînement (29) pourvu d'un contour extérieur approprié, qui s'engage dans l'évidement du déplaceur lors du couplage de l'unité d'entraînement.

6. Dispositif selon l'une quelconque des revendications 1 à 5, **caractérisé en ce que** la partie de boîtier (4) recevant la chambre de pompe (12) présente deux connecteurs (13, 14) pour le raccordement d'un flexible d'alimentation à l'entrée (11) et la sortie (10) de la chambre de pompe.

7. Flexible d'alimentation pour le transfert d'une solution nutritive pour l'alimentation entérale d'un sachet (29) contenant la solution nutritive à une sonde (32), qui présente un flexible de sonde (33) à introduire dans l'estomac du patient, **caractérisé par** un dispositif (1) branché sur le flexible d'alimentation (30, 31) pour le dosage de la solution nutritive selon l'une quelconque des revendications 1 à 6.

8. Dispositif pour l'alimentation entérale avec un sachet (29) contenant la solution nutritive, un flexible d'alimentation (30, 31) et une sonde (32), qui présente un flexible de sonde (33) à introduire dans l'estomac du patient, **caractérisé par** un dispositif (1) branché sur le flexible d'alimentation (30, 31) pour le dosage de la solution nutritive selon l'une quelconque des revendications 1 à 6.
